# EUROPEAN PATENT SPECIFICATION

(11) **EP 3 596 196 B1**
(45) Date of publication and mention of the grant of the patent: **05.05.2021**
(21) Application number: 18755215.3
(22) Date of filing: 15.03.2018
(51) Int. Cl.: C12M 1/26, A61K 35/35, C12M 1/33, C12M 1/00

(54) **FILTER APPARATUS**
FILTERVORRICHTUNG
APPAREIL DE FILTRE

(30) Priority: 17.03.2017 FI 20175238
(43) Date of publication of application: 22.01.2020
(73) Proprietor: Everfill Oy, 00120 Helsinki (FI)
(72) Inventor: SARKANEN, Jertta-Riina, 33014 Tampereen Yliopisto (FI); YLIKOMI, Timo, 33014 Tampereen Yliopisto (FI); LOPEZ, Jenny, 33014 Tampereen Yliopisto (FI); SEPPO, Matti, 33014 Tampereen Yliopisto (FI)
(74) Representative: Kolster Oy Ab
(86) International application number: PCT/FI2018/050191
(87) International publication number: WO 2018/167371

(56) References cited:
- WO-A1-2016/015767
- US-A1- 2005 260 175
- US-A1- 2013 344 589
- US-A1- 2016 333 305

## Description

### FIELD OF THE INVENTION

The present invention relates to soft tissue engineering. More particularly, the present invention is directed to a method of separating and collecting an adipose tissue extract and adipose tissue from a fat sample, and a filter apparatus for separating and collecting an adipose tissue extract and an adipose tissue from a fat sample.

### BACKGROUND

Soft tissue consists of connecting and supporting structures in the body such as muscles, connective tissue, adipose tissue and blood vessels. Soft tissue engineering seeks to fabricate replacement parts for soft tissue defects resulting from e.g. trauma (burns and scars), surgical resection or con-genital malformations. In addition, cosmetic use, such as filling of facial wrinkles, is an important application of reconstituted soft tissue.

Alloplastic materials, such as silicon and bovine collagen, have been used in tissue engineering. Such materials may, however, cause severe rejection reactions, as well as allergic reactions. Thus, the use of natural transplant materials is nowadays preferred.

Autologous transplants would be desirable for use in soft tissue engineering. Autologous adipose tissue transplantation is an old therapeutic procedure, where mature adipocytes or adipose tissue itself are transplanted into the site of defect. Adipose tissue is abundant, easy to collect, and readily obtainable for clinical uses. However, the use of autologous adipose tissue transplants may still result in several problems such as resorption, excessive connective tissue (scar) formation, inflammation reactions, as well as hardening and clotting of the transplant. Furthermore, long-term results obtained with adipose tissue transplantation are unpredictable and variable, depending on the method used and the skills of the person executing said method.

Adipose stem cells have been used for soft tissue engineering. Such cells are capable of proliferating and differentiating into mature adipose tissue. However, as the extracellular matrix has an essential role in cell proliferation and differentiation, it is unlikely that repair of large or deep soft tissue defects would be possible with cells alone. In order to be regenerated into functional tissue, transplanted cells need an artificial extracellular matrix, i.e. biomaterial scaffold to aid in cell attachment, proliferation and differentiation.

Formation of new blood vessels, i.e. neovascularization, is crucial for nutrient supply and waste disposal in the regenerating soft tissue. Single growth factors (e.g. FGF-2 or VEGF) have been used as bioactive substances for this purpose, but the results have not been satisfactory. Using a pool of bioengineered growth factors in a single implant would result in very high costs. US 20130344589 A1 describes a filtering device comprising two flat filters to separate adipose tissue extract.

A cellfree adipose tissue extract has been found useful for many purposes, but to date, no adequate method and filter apparatus has been available for separating the cellfree adipose tissue extract and adipose tissue in the fat sample in a manner enabling sterile preparation and optimal reuse of both. Therefore, the production of particularly such adipose tissue extract has been laborious and it has required a lot of manual work phases.

### BRIEF DESCRIPTION

An object of the present invention is thus to provide a new method and an apparatus for separating and collecting adipose tissue extract and adipose tissue from a fat sample. The objects of the invention are achieved by a method and an arrangement, which are characterized by what is stated in the independent claims. Preferred embodiments of the invention are disclosed in the dependent claims.

The invention is based on the idea of providing a new kind of a filter for filtering components of the fat sample and a filter apparatus comprising such a filter.

An advantage of the method and arrangement of the invention is that large volumes of adipose tissue can be filtered and the properties of the adipose tissue extract can be optimized by a suitable filtering pace. The method and the apparatus enable collecting both the adipose tissue and the adipose tissue extract in a sterile manner from a fat sample provided for instance by liposuction, whereby the adipose tissue comprising adipose tissue pieces and adipose tissue cells and the adipose tissue extract are both usable for instance for use as transplants or other suitable purposes. Other advantages of the solution are explained in connection with the description.

### BRIEF DESCRIPTION OF THE DRAWINGS

In the following the invention will be described in greater detail by means of preferred embodiments with reference to the attached drawings, in which
Figure 1 is an exploded view of an embodiment of a filter element;
Figure 2 is an exploded view of another embodiment of a filter element;
Figure 3 illustrates a filter apparatus; and
Figure 4 illustrates a method for separating an adipose tissue extract and adipose tissue from fat sample.

### DETAILED DESCRIPTION OF THE INVENTION

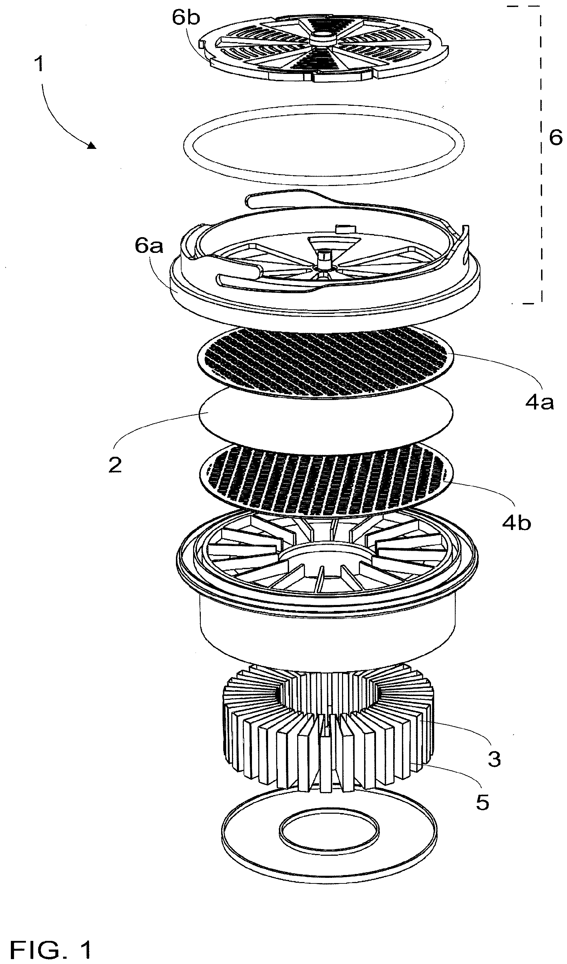

Figure 1 is an exploded view of an embodiment of a filter element 1 for separating an adipose tissue extract and adipose tissue from fat sample. In this description adipose tissue extract and adipose tissue refer to the components of a fat sample. Fat sample can comprise for instance a liposuction sample, a fat tissue sample (tissue block) that may have been first minced or cut into small pieces and possibly also incubated for instance for 15 minutes to 24 hours, or any other adipose tissue derived disintegrated tissue sample. Adipose tissue refers to the solid particles of the fat sample, comprising adipose tissue pieces and different types of cells, such as stem cells, endothelium cells, macrophages and the so on. Adipose tissue extract, on the other hand, refers to the liquid cellfree extract extracted from the fat sample by separating the adipose tissue. The adipose tissue extract can, thus, especially in connection with a liposuction sample also be called liposuction liquid. Liposuction samples may typically comprise a product of liposuction collected during an operation, for example. According to an embodiment, the adipose tissue is separated from the fat sample by filtering carried out in a controlled manner, whereby the liquid residue from which the adipose tissue has been removed forms the so-called adipose tissue extract.

The filter element 1 comprises a first filter 2. According to an embodiment, the first filter 2 may comprise fiberglass. According to another embodiment, the first filter 2 may comprise in addition to or instead of fiberglass at least one of the following: cellulose acetate, polysulfone, polyether sulfone, polyacrilonitrile, polyvinylidiene flouride, polypropylene, polyethylene, and polyvinyl chloride. According to other embodiments, the first filter 2 may comprise some other suitable material providing the effects described in this description.

The filter element 1 further comprises a second filter 3. According to an embodiment, the second filter 3 may comprise polyether sulfone. According to another embodiment, the second filter 3 may comprise in addition to or instead of polyether sulfone at least one of the following: cellulose acetate, polysulfone, polyether sulfone, polyacrilonitrile, polyvinylidiene flouride, polypropylene, polyethylene, and polyvinyl chloride. According to other embodiments, the first filter 2 may comprise some other suitable material providing the effects described in this description.

Both the first filter 2 and the second filter 3 are formed to pass through adipose tissue extract but not pass through adipose tissue. Therefore, such a filter element 1 can be used for separating the components of the fat sample, namely adipose tissue extract that is capable of passing through the first filter and the second filter and adipose tissue that is not capable of passing through neither of the first filter and the second filter, from one another. The first filter 2 and the second filter 3 may be arranged in the filter element in such a manner that an adipose tissue extract passing through the filter element 1 can be brought into contact with the first filter 2 before it is brought into contact with the second filter 3.

According to an embodiment, the first filter 2 may be formed as a flat, sheet-like piece. A benefit of this embodiment is that it is simpler to manufacture and a suitable pore size ensures both a good quality filtering and a sufficient flow through rate. In some other embodiments, the first filter 2 may also comprise a cylindrical, folded and/or any other suitable form to increase the filter surface area of the first filter 2, for example.

According to an embodiment, the first filter 2 may comprise a pore size in the range of 0.5 to 2 µm, preferably in the range of 0.7 to 1.2 µm, and most preferably in the range of 0.8-1.1 µm. In such embodiments, the first filter 2 may provide a preliminary filter that stops solid materials that might block the second filter 3 from coming into contact with the second filter 3. According to an embodiment, the second filter 3 may comprise a pore size in the range of 0.1 to 0.5 µm, preferably in the range of 0.15 to 0.3 µm, and most preferably in the range of 0.2 to 0.22 µm to optimize the filtering.

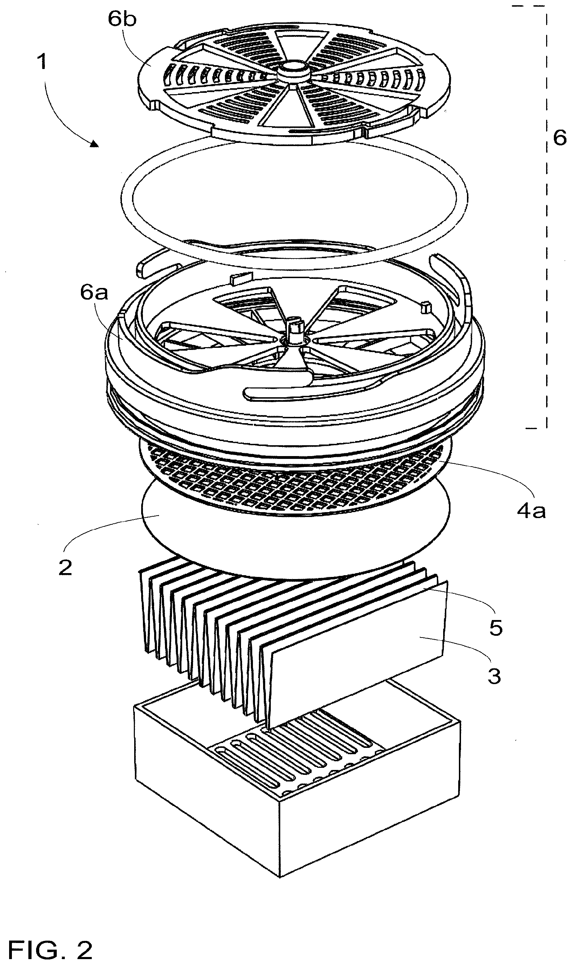

According to an embodiment, there may be provided a support mesh 4a, 4b at least on one side of the first filter 2, namely on the second filter side or on the side the opposite to the second filter of the first filter 2, to support the first filter. According to an embodiment, there may be provided a support mesh 4a, 4b on both sides of the first filter 2, namely on the second filter side and on the opposite side of the first filter 2, to support the first filter. Figure 1 shows an example of an embodiment with two support meshes 4a, 4b, one on each side of the first filter 2, and Figure 2 shows an example of an embodiment with one support mesh 4a. However, it is clear for a person skilled in the art that the embodiments of Figures 1 and 2 are only shown to illustrate some embodiments of a filter element 1. Thus, in other embodiments a filter element otherwise similar to that of Figure 2 might also comprise two support meshes 4a, 4b, and a filter element otherwise similar to that of Figure 1 might only comprise one support mesh 4a, for example. The use of the mesh(es) provides support for the first filter 2, whereby a first filter 2 with a suitable filtering pace and a more compact structure can be provided. According to a further embodiment, the mesh(es) 4a, 4b may comprise a plastic material. According to a yet another embodiment, the mesh(es) 4a, 4b may comprise instead of or in addition to a plastic material at least one of the following: a metallic material and a ceramic material. In other words, the mesh(es) may also comprise a combination of these materials. According to a yet another embodiment, the mesh(es) 4a, 4b may comprise a yet another material or a combination of materials that can also be sterilized by gamma irradiation or autoclaving. Depending on the embodiment the mesh(es) 4a, 4b may be provided as separate structural parts or one or both of them may be integrated with the first filter 2 and/or other parts of the filter element 1.

According to an embodiment, the second filter 3 may comprise a flat sheet-like structure. According to an embodiment, the second filter 3 may comprise the sheet-like structure folded in an accordion-like manner with the folds 5 extending in either horizontal or in vertical direction. The second filter 3 may be formed as a cylindrical piece that may, depending on the embodiment, be solid or hollow in a pipe-like formation. According to a yet another embodiment, the second filter 3 formed as a cylindrical piece may also comprise folds 5 extending in a horizontal direction, as disclosed in Figure 1. A folded form is particularly beneficial for the second filter 3 with smaller pore size as it increases the filter surface area of the second filter 3. Figure 1 shows an embodiment with folds 5 extending in vertical direction in a cylindrical formation and Figure 2 shows an embodiment with folds 5 extending in horizontal direction.

According to the invention, the filter element 1 further comprises a partition element 6 provided on the opposite side of the first filter 2 compared to the side of the second filter 3. The partition element 6 has at least two use positions. In the first use position of the partition element the partition element closes the connection between the first filter 2 and the outside of the filter element 1. In the second use position of the partition element a connection between the first filter 2 and the outside of the filter element 1 is provided, such that the first filter is enabled to come into contact with a substance entering the filter element from outside of the filtering element for filtering the substance. This enables controlling when the filtering of the fat sample brought into contact with the filter element 1 is started.

According to the invention, the partition element 6 comprises a two-layer structure, wherein a first layer 6a and a second layer 6b of the partition element both comprise apertures 7. The first layer 6a and the second layer 6b are rotatable with respect to one another. According to an embodiment, the first layer 6a may be arranged immovable with respect to the first filter 2 and the second filter 3, and the second layer 6b may arranged rotatably with respect to first layer 6a and, thereby, to the first filter 2 and the second filter 3.

In the first use position of the partition element the apertures 7 of the first layer 6a and the second layer 6b may not be overlapping. In other words each layer 6a, 6b may cover the apertures in the other layer, such that the layers form a uniform cover without apertures for the first filter 2. In the second use position of the partition element the apertures 7 of the first layer 6a and the second layer 6b may be at least partly overlapping, such that apertures extending through the partition element are provided. In other words, the overlapping parts of the apertures 7 of the different layers aligned on top of one another together provide a connection between the interior and the exterior of the filter element 1.

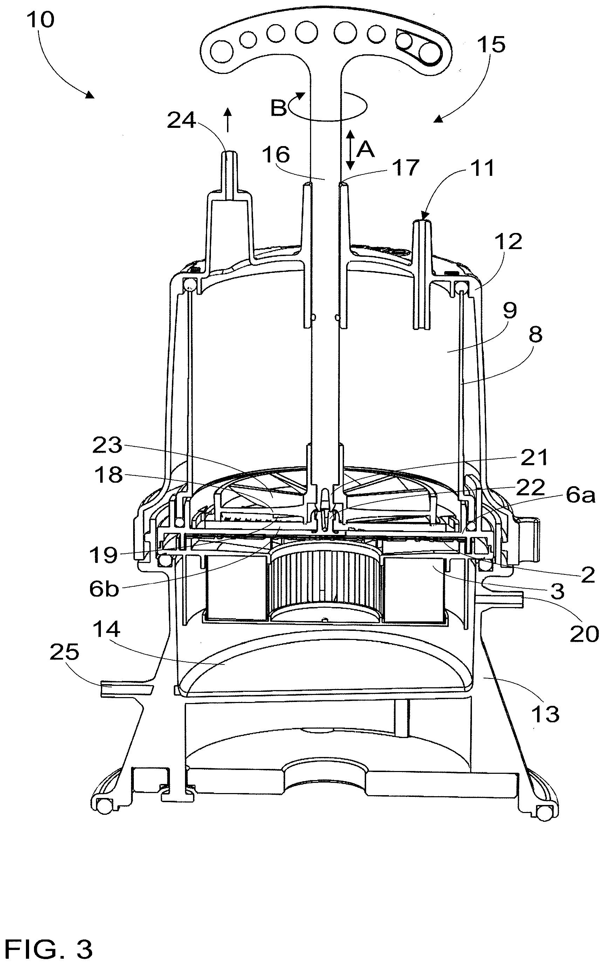

Figure 3 illustrates a filter apparatus 10 for separating an adipose tissue extract and adipose tissue from fat sample. The filter apparatus 10 comprises a first receptacle 8 comprising a first space 9 for holding adipose tissue. The filter apparatus 10 further comprises a second receptacle 13 comprising a second space 14 for receiving adipose tissue extract. An advantage of such a filter apparatus is that handling of a fat sample, such as a liposuction sample, can be done in a controlled and sterile manner for example in operating room environment.

The filter apparatus 10 further comprises a first orifice 11 for receiving fat sample in the first receptacle 8. According to an embodiment, the first orifice 11 may be provided in the first receptacle 8. According to such an embodiment the first receptacle may be formed as one piece, for example. According to another embodiment, the receptacle may comprise a lid 12 and the first orifice 11 may be provided in the lid 12, such as in the embodiment of Figure 3. According to an embodiment, the lid may be removably connected to the first receptacle 8, the second receptacle 13 or other structural part of the filter apparatus 10 in manner known as such, such as by means of threading.

The filter apparatus 10 further comprises a filter element 1, such as a filter element according to Figure 1 or Figure 2 and/or according to an embodiment disclosed in this description. The filter element 1 is provided between the first space 9 and the second space 14. The filter element 1 is arranged to separate the adipose tissue extract and the adipose tissue by passing through the adipose tissue extract to enter the second receptacle 13 and by not passing through the adipose tissue, whereby the adipose tissue is kept in the first receptacle 8.

According to an embodiment, the filter apparatus 10 may further comprise a partition element 6 provided between the first space 9 and the first filter 2. This partition element 6 may comprise a partition element disclosed in connection with the filter element embodiments and/or in connection with the embodiments of Figures land 2. The partition element 6 may have at least two use positions. In the first use position of the partition element, the partition element 6 may isolate the first space 9 and the first filter 2. In other words, the partition element 6 may isolate the first space 9 and the first filter 2 from one another. The partition element 6 thus, at the same time, isolates the first space 9 from the second filter 3 and/or the second receptacle 13. In the second use position of the partition element there may be provided a connection between the first space 9 and the first filter 2, such that the content of the first space 9 is enabled to come into contact with the first filter 2 and the second filter 3 for filtering the content. An advantage of such embodiments is that the time the fat sample is kept in the first space 9 before starting the filtering process can be controlled and optimized and, therefore, also the composition of the end products, namely the adipose tissue extract and adipose tissue, can be controlled and optimized for an intended use.

According to an embodiment, the partition element 6, such as a partition element of one of the embodiments disclosed in connection with Figures 1, 2 and/or 3, may be formed in such a manner that the change of the use position of the partition element 6 is irreversible. In other words, the partition element 6 may be formed in such a manner that once the partition element has been arranged to the second use position it cannot be brought back to the first use position. Different ways of arranging such irreversible change are generally known and are therefore not disclosed in more detail, but this may comprise for example providing mechanical parts that cannot be disengaged once they have been engaged or by providing one of the parts with a protrusion protruding to an aperture 7 once aligned with it and prohibiting the return of the partition element to the first use position. An advantage of such embodiments is that the partition part, the filter element and/or the filter apparatus cannot be accidentally used for a second time.

According to the invention, the first filter 2 and the second filter 3 are arranged in the filter apparatus 10 in such a manner that adipose tissues extract transferring from the first space 9 to the second space 14 is brought into contact with the first filter 2 before it is brought into contact with the second filter 3.

According to an embodiment, the filter apparatus 10 may be provided with an insert part 15. The insert part 15 may comprise a shaft 16 movably connected to the first receptacle 8. The shaft 16 may comprise an outer portion extending to the outside of the first receptacle through a sealed shaft opening 17 and an inner portion provided inside the first space 9.

The insert part 15 may further comprise stirring means 18 for stirring the fat sample in the first space 9. The stirring means 18 may comprise a structure capable of stirring fat sample, such as beater- or propeller-like structure. According to an embodiment, the stirring means may comprise a propeller-like construction comprising blades 23 for stirring the content. According to a further embodiment, the blades 23 may be provided at an angle compared to the longitudinal direction A of the shaft for improved stirring of the content, such as the fat sample.

According to an embodiment, the insert part 15 may further comprise opening means 19 for engaging with at least part of the partition element 6 for changing the use position of the partition element from the first use position to the second use position of the partition element. The opening means 19 may for instance comprise an engaging part and the partition element 6 may comprise a counterpart, wherein the engaging part 21 of the insert part 15 and the counterpart 22 of the partition element 6 are formed to provide form-locking between the insert part 15 and the partition element 6. According to an embodiment, the opening means 19 may be irreversibly engageable with the partition element 6. In other words, the opening means may be engaged with the partition element in such a manner that they cannot be disengaged in the course of intended use of the filter element 1 and/or the filter apparatus 10. For instance, the form-locking between the engaging part 21 and the counterpart 22 may be formed irreversible in the course of intended use of the filter element 1 and/or the filter apparatus 10.

The outer portion of the shaft 16 may be seizable for moving the insert part 15 in the longitudinal direction A of the shaft and for turning the insert part 15 about the longitudinal direction of the shaft, in other words in the direction B, to stir the content of the first space 9 and/or to engage with the partition element 6 to change its use position. According to an embodiment, the outer portion may comprise a handle or other type of gripping means for seizing the shaft 16 and, thereby, the insert part 15 for moving it. According to an embodiment, the outer portion of the shaft may be connectable to a motor or other type of an actuator for moving the insert part 15 in direction A and/or direction B. This enables automated use of the filter apparatus 10.

According to an embodiment, the second space 14 may be connected to a third orifice 20 for connecting the second space 14 to vacuum to provide suction for improved filtering of adipose tissue extract when compared with filtering based on force of gravity only.

According to an embodiment, the first receptacle 8 and/or the lid 12 may be provided with a fourth orifice 24 for connecting with vacuum for providing a low pressure in the first receptacle for instance for sucking content, such as fat sample, to the first receptacle.

According to an embodiment, the third orifice 20, the fourth orifice 24 and/or any other air outlet orifice of the filter apparatus 10 may be provided with HEPA filter or some other suitable filter with a pore size of 2 µm or smaller for providing a completely closed system.

According to an embodiment, the second space 14 may further be connected to a fifth orifice 25 for removing the filtered extract, such as adipose filter extract, from the second receptacle 13. This is particularly beneficial if the adipose tissue extract is used during the same operation in connection with which fat sample, such as a liposuction sample, has been collected, since this enables sterile handling of the adipose tissue extract during all phases of the filtering process.

According to an embodiment, the bottom of filter apparatus 10 may be provided with a material increasing the friction between the filter apparatus 10 and the surface it is placed on, such as a rubber ring. The bottom of the filter apparatus 10 may also be formed wider than the upper parts of the filter apparatus and/or the filter apparatus 10 may be provided with thicker and/or denser material and/or a separate weight structure at the bottom part of the filter apparatus 10 to improve its stability.

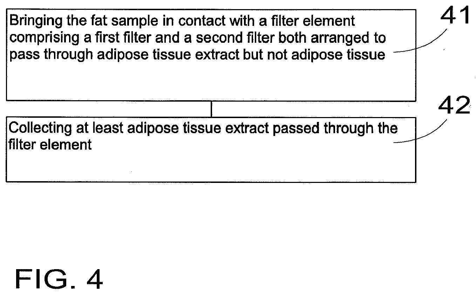

Figure 4 illustrates a method for separating an adipose tissue extract and adipose tissue from a fat sample. The method may comprise bringing 41 the fat sample in contact with a filter element 1 according to at least one of the embodiments disclosed in this description or a combination thereof. In other words, the method may comprise first bringing the fat sample in contact with a first filter 2 of the filter element 1 and then bringing the fat sample in contact with a second filter 3 of the filter element 1 according to at least one of the embodiments disclosed in this description or a combination thereof. Both the first filter and the second filter may pass through adipose tissue extract but not adipose tissue. The method may further comprise collecting 42 at least adipose tissue extract passed through the filter element 1. According to an embodiment, the method may further comprise collecting adipose tissue that has not passed through the filter element 1.

According to an embodiment, the method may further comprise providing the fat sample in a first space 9 of a filter apparatus 10 according to at least one of the embodiments disclosed in this description or a combination thereof. The method may further comprise changing the use position of the partition element 6 for bringing the fat sample in contact with the filter element 1, collecting the adipose tissue extract passed through the filter element 1 from the second space 14, and/or collecting the adipose tissue not passed through the filter element from the first space 9.

According to an embodiment, the method may further comprise at least one functional step disclosed in this description or a combination thereof. For instance, the filter apparatus 10 may comprise a partition element 6; and the method may comprise isolating by the partition element 6 the first space 9 and the first filter 2 from one another in a first use position of the partition element and providing in a second use position of the partition element a connection between the first space 9 and the first filter 2, such that the content of the first space 9 is enabled to come into contact with the first filter 2 and the second filter 3 for filtering the content.

Filter elements 1 and filter apparatuses 10 disclosed in this description are scalable depending on the specific needs of different use situation and enable for instance providing a closed and sterile system capable of filtering large volumes of content to be filtered, such as a fat sample. During an operation, for example, there may be a need for filter amounts such as 400 to 500 ml of the fat sample. On the other hand, filter elements and filter apparatuses of different filter paces and/or of different volumes may be useful for different types of purposes and use situations. For instance, according to an embodiment, the filter element or filter apparatus may be suitable for filtering fat samples of smaller than or equal to 50 ml. In such embodiments with very small volumes required, the filter element 1 may also be provided in a syringe instead of the filter apparatus 10. According to another embodiment, the filter element or filter apparatus may be suitable for filtering fat samples of 400 to 500 ml. According to a yet another embodiment, the filter element or filter apparatus may be suitable for filtering fat samples of 3 to 4 litres, in some cases even more. Additionally, the filter elements and filter apparatuses of this description enable good control and quality of the composition of the filtered extract, such as adipose tissue extract as the filtering process can be controlled.

## Claims

1. A filter element (1) for separating an adipose tissue extract and adipose tissue from a fat sample, wherein the filter element comprises a first filter (2) and a second filter (3), wherein through both first filter and second filter adipose tissue extract passes but adipose tissue does not pass, and a partition element (6) having at least two use positions, wherein in the first use position of the partition element the partition element closes the connection between the first filter and the outside of the filter element and in the second use position of the partition element there is provided a connection between the first filter and the outside of the filter element, such that the first filter is enabled to come into contact with a substance entering the filter element from outside of the filtering element for filtering the substance, wherein the partition element comprises a two-layer structure, wherein a first layer (6a) and a second layer (6b) of the partition element both comprise apertures (7) and are rotatable with respect to one another, and wherein in the first use position of the partition element the apertures of the first layer and the second layer are not overlapping, such that each layer covers the apertures in the other layer, and in the second use position of the partition element the apertures are at least partly overlapping, such that apertures extending through the partition element are provided, and
wherein the partition element is arranged in the filter element in such a manner that the substance can be brought into contact with the partition element before the first filter and the first filter and the second filter are arranged in the filter element in such a manner that an adipose tissue extract passing through the filter element can be brought into contact with the first filter before it is brought into contact with the second filter.

2. A filter element according to claim 1, wherein the first filter is formed as one of the following: a flat, sheet-like piece; a cylindrical piece; and a folded horizontal piece; and/or the second filter comprises a flat sheet-like structure or a sheet-like structure folded in an accordion-like manner with the folds extending in either horizontal or in vertical direction.

3. A filter element according to claim 1 or 2, wherein the first filter comprises at least one of the following: fiberglass, cellulose acetate, polysulfone, polyether sulfone, polyacrilonitrile, polyvinylidiene flouride, polypropylene, polyethylene, and polyvinyl chloride; and/or
the second filter comprises at least one of the following: polyether sulfone, cellulose acetate, polysulfone, polyether sulfone, polyacrilonitrile, polyvinylidiene flouride, polypropylene, polyethylene, and polyvinyl chloride.

4. A filter apparatus (10) for separating an adipose tissue extract and adipose tissue from a fat sample, the filter apparatus comprising
a first receptacle (8) comprising a first space (9) for holding adipose tissue, a first orifice (11) for receiving a fat sample in the first receptacle, a second receptacle (13) comprising a second space (14) for receiving adipose tissue extract, and
provided between the first space and the second space a filter element according to any one of claims 1-3 for separating the adipose tissue extract and the adipose tissue by passing through the adipose tissue extract to enter the second receptacle and by not passing through the adipose tissue, whereby the adipose tissue is kept in the first receptacle.

5. A filter apparatus according to claim 4, wherein a partition element is provided between the first space and the first filter and has at least two use positions, wherein in the first use position of the partition element the partition element isolates the first space and the first filter and in the second use position of the partition element there is a connection between the first space and the first filter such that the content of the first space is enabled to come into contact with the first filter and second filter for filtering the content.

6. A filter apparatus according to claim 4 or 5, wherein the filter apparatus is provided with an insert part (15) comprising a shaft (16) movably connected to the first receptacle, the shaft comprising an outer portion extending to the outside of the first receptacle through a sealed shaft opening and an inner portion provided inside the first space,
stirring means (18) for stirring the fat sample in the first space, and opening means (19) for engaging with at least part of the partition element for changing the use position of the partition element from the first use position to the second use position of the partition element,
wherein the outer portion of the shaft is seizable for moving the insert part in the longitudinal direction of the shaft and for turning the insert part about the longitudinal direction of the shaft to stir the content of the first space and to engage with the partition element to change its use position.

7. A method for separating an adipose tissue extract and adipose tissue from a fat sample, the method comprising
bringing the fat sample in contact with a filter element of any one of the claims 1-3 and
collecting at least adipose tissue extract passed through the filter element.

8. A method according to claim 7, the method further comprising
providing the fat sample in a first space of a filter apparatus according to claim 5 or 6,
changing the use position of the partition element for bringing the fat sample in contact with the filter element,
collecting the adipose tissue extract passed through the filter element from the second space, and
collecting the adipose tissue not passed through the filter element from the first space.

## Patentansprüche

1. Filterelement (1) zum Trennen eines Fettgewebeextrakts und von Fettgewebe aus einer Fettprobe, wobei das Filterelement Folgendes umfasst
ein erstes Filter (2) und
ein zweites Filter (3),
wobei Fettgewebeextrakt sowohl das erste Filter als auch das zweite Filter passiert, nicht aber des Fettgewebe dieselben passiert, und
ein Trennelement (6) mit mindestens zwei Gebrauchspositionen, wobei in der ersten Gebrauchsposition des Trennelements das Trennelement die Verbindung zwischen dem ersten Filter und der Außenseite des Filterelements schließt und in der zweiten Gebrauchsposition des Trennelements eine Verbindung zwischen dem ersten Filter und der Außenseite des Filterelements bereitgestellt ist, derart, dass es dem ersten Filter möglich ist, mit einer Substanz, die von außerhalb des Filterelements in das Filterelement eindringt, zum Filtern der Substanz in Kontakt zu kommen, wobei das Trennelement eine Zweischichtstruktur umfasst, wobei eine erste Schicht (6a) und eine zweite Schicht (6b) des Trennelements beide Öffnungen (7) umfassen und mit Bezug aufeinander drehbar sind,
und wobei in der ersten Gebrauchsposition des Trennelements die Öffnungen der ersten Schicht und der zweiten Schicht nicht überlappen, derart, dass jede Schicht die Öffnungen in der anderen Schicht abdeckt, und in der zweiten Gebrauchsposition des Trennelements die Öffnungen mindestens teilweise überlappen, derart, dass Öffnungen bereitgestellt sind, die sich durch das Trennelement erstrecken, und
wobei das Trennelement in einer Weise im Filterelement angeordnet ist, in der die Substanz vor dem ersten Filter mit dem Trennelement in Kontakt gebracht werden kann, und das erste Filter und das zweite Filter in einer Weise im Filterelement angeordnet sind, in der ein Fettgewebeextrakt, das das erste Filterelement passiert, mit dem ersten Filter in Kontakt gebracht werden kann, bevor er mit dem zweiten Filter in Kontakt gebracht wird.

2. Filterelement nach Anspruch 1, wobei das erste Filter als eines von Folgendem gebildet ist: ein flaches plattenförmiges Stück; ein zylindrisches Stück und ein gefaltetes horizontales Stück; und/oder das zweite Filter eine flache plattenförmige Struktur oder eine plattenförmige Struktur, die in einer Akkordeonweise gefaltet ist, wobei sich die Falten entweder in die horizontale oder in die vertikale Richtung erstrecken, umfasst.

3. Filterelement nach Anspruch 1 oder 2, wobei das erste Filter mindestens eines von Folgendem umfasst: Glasfasern, Celluloseacetat, Polysulfon, Polyethersulfon, Polyacrylnitril, Polyvinylidienfluorid, Polypropylen, Polyethylen und Polyvinylchloride und/oder
das zweite Filter mindestens eines von Folgendem umfasst: Polyethersulfon, Celluloseacetat, Polysulfon, Polyethersulfon, Polyacrylnitril, Polyvinylidienfluorid, Polypropylen, Polyethylen und Polyvinylchlorid.

4. Filtervorrichtung (10) zum Trennen eines Fettgewebeextrakts und von Fettgewebe aus einer Fettprobe, wobei die Filtervorrichtung Folgendes umfasst
einen ersten Behälter (8), der einen ersten Raum (9) zum Enthalten von Fettgewebe umfasst,
eine erste Durchführung (11) zum Aufnehmen einer Fettprobe im ersten Behälter,
einen zweiten Behälter (13), der einen zweiten Raum (14) zum Aufnehmen eines Fettgewebeextrakts umfasst, und
ein zwischen dem ersten Raum und dem zweiten Raum bereitgestelltes Filterelement nach einem der Ansprüche 1-3 zum Trennen des Fettgewebeextrakts und des Fettgewebes durch Passieren des Fettgewebeextrakts, um in den zweiten Behälter einzutreten, und durch Nichtpassieren des Fettgewebes, wodurch das Fettgewebe im ersten Behälter gehalten wird.

5. Filtervorrichtung nach Anspruch 4, wobei ein Trennelement zwischen dem ersten Raum und dem ersten Filter bereitgestellt ist und mindestens zwei Gebrauchspositionen aufweist, wobei in der ersten Gebrauchsposition des Trennelements das Trennelement den ersten Raum und das erste Filter isoliert und in der zweiten Gebrauchsposition des Trennelements eine Verbindung zwischen dem ersten Raum und dem ersten Filter besteht, derart, dass es dem Inhalt des ersten Raums möglich ist, zum Filtern des Inhalts mit dem ersten Filter und dem zweiten Filter in Kontakt zu kommen.

6. Filtervorrichtung nach Anspruch 4 oder 5, wobei die Filtervorrichtung mit einem Einsatzteil (15) versehen ist, das Folgendes umfasst
eine Welle (16), die bewegbar mit dem ersten Behälter verbunden ist, wobei die Welle einen Außenabschnitt umfasst, der sich durch eine versiegelte Wellenöffnung nach außerhalb des ersten Behälters erstreckt, und einen Innenabschnitt, der im ersten Raum bereitgestellt ist,
ein Rührmittel (18) zum Rühren der Fettprobe im ersten Raum, und
ein Öffnungsmittel (19) zum mindestens teilweisen Eingreifen in das Trennelement zum Ändern der Gebrauchsposition des Trennelements von der ersten Gebrauchsposition in die zweite Gebrauchsposition des Trennelements,
wobei der Außenabschnitt der Welle zum Bewegen des Einsatzteils in die Längsrichtung der Welle und zum Drehen des Einsatzteils um die Längsrichtung der Welle greifbar ist, um den Inhalt des ersten Raums zu rühren und um in das Trennelement einzugreifen, um dessen Gebrauchsposition zu ändern.

7. Verfahren zum Trennen eines Fettgewebeextrakts und von Fettgewebe aus einer Fettprobe, wobei das Verfahren Folgendes umfasst
Inkontaktbringen der Fettprobe mit einem Filterelement von einem der Ansprüche 1-3 und
Sammeln von mindestens Fettgewebeextrakt, der durch das Filterelement passiert wird.

8. Verfahren nach Anspruch 7, wobei das Verfahren ferner Folgendes umfasst
Bereitstellen der Fettprobe in einem ersten Raum einer Filtervorrichtung nach Anspruch 5 oder 6,
Ändern der Gebrauchsposition des Trennelements, um die Fettprobe mit dem Filterelement in Kontakt zu bringen,
Sammeln des Fettgewebeextrakts, der durch das Filterelement passiert wird, aus dem zweiten Raum, und
Sammeln des Fettgewebes, der durch das Filterelement passiert wird, aus dem ersten Raum.

## Revendications

1. Élément de filtre (1) pour séparer un extrait de tissu adipeux et un tissu adipeux d'un échantillon de graisse, dans lequel l'élément de filtre comprend :
un premier filtre (2), et
un second filtre (3),
dans lequel l'extrait de tissu adipeux passe mais le tissu adipeux ne passe pas à travers le premier filtre et le second filtre, et
un élément de séparation (6) ayant au moins deux positions d'utilisation, dans lequel dans la première position d'utilisation de l'élément de séparation, l'élément de séparation ferme le raccordement entre le premier filtre et l'extérieur de l'élément de filtre et dans la seconde position d'utilisation de l'élément de séparation, on prévoit un raccordement entre le premier filtre et l'extérieur de l'élément de filtre, de sorte que le premier filtre est apte à venir en contact avec une substance entrant dans l'élément de filtre depuis l'extérieur de l'élément de filtration pour filtrer la substance, dans lequel l'élément de séparation comprend une structure à deux couches, dans lequel une première couche (6a) et une seconde couche (6b) de l'élément de séparation comprennent toutes deux des ouvertures (7) et peuvent tourner l'une par rapport à l'autre, et dans lequel, dans la première position d'utilisation de l'élément de séparation, les ouvertures de la première couche et de la seconde couche ne se chevauchent pas de sorte que chaque couche recouvre les ouvertures de l'autre couche, et dans la seconde position d'utilisation de l'élément de séparation, les ouvertures se chevauchent au moins partiellement, de sorte que des ouvertures s'étendant à travers l'élément de séparation sont fournies, et
dans lequel l'élément de séparation est agencé dans l'élément de filtre de sorte que la substance peut être amenée en contact avec l'élément de séparation avant le premier filtre, et le premier filtre et le second filtre sont agencés dans l'élément de filtre de sorte qu'un extrait de tissu adipeux traversant l'élément de filtre peut être amené en contact avec le premier filtre avant qu'il soit amené en contact avec le second filtre.

2. Élément de filtre selon la revendication 1, dans lequel le premier filtre est formé comme l'un des éléments suivants : une pièce plate en forme de feuille ; une pièce cylindrique ; et une pièce horizontale pliée ; et/ou le second filtre comprend une structure plate en forme de feuille ou une structure en forme de feuille pliée en forme d'accordéon avec les plis qui s'étendent dans la direction horizontale ou verticale.

3. Élément de filtre selon la revendication 1 ou 2, dans lequel le premier filtre comprend au moins l'un des éléments suivants : de la fibre de verre, de l'acétate de cellulose, du polysulfone, du polyéther sulfone, du polyacrylonitrile, du fluorure de polyvinylidène, du polypropylène, du polyéthylène et du chlorure de polyvinyle ; et/ou
le second filtre comprend au moins l'un des éléments suivants : du polyéther sulfone, de l'acétate de cellulose, du polysulfone, du polyéther sulfone, du polyacrylonitrile, du fluorure de polyvinylidène, du polypropylène, du polyéthylène et du chlorure de polyvinyle.

4. Appareil de filtre (10) pour séparer un extrait de tissu adipeux et un tissu adipeux d'un échantillon de graisse, l'appareil de filtre comprenant :
un premier réceptacle (8) comprenant un premier espace (9) pour contenir le tissu adipeux,
un premier orifice (11) pour recevoir un échantillon de graisse dans le premier réceptacle,
un second réceptacle (13) comprenant un second espace (14) pour recevoir l'extrait de tissu adipeux, et
prévu entre le premier espace et le second espace, un élément de filtre selon l'une quelconque des revendications 1 à 3 pour séparer l'extrait de tissu adipeux et le tissu adipeux en traversant l'extrait de tissu adipeux pour entrer dans le second réceptacle, et en ne traversant pas le tissu adipeux, moyennant quoi le tissu adipeux est maintenu dans le premier réceptacle.

5. Appareil de filtre selon la revendication 4, dans lequel un élément de séparation est prévu entre le premier espace et le premier filtre et a au moins deux positions d'utilisation, dans lequel, dans la première position d'utilisation de l'élément de séparation, l'élément de séparation isole le premier espace et le premier filtre et dans la seconde position d'utilisation de l'élément de séparation, il y a un raccordement entre le premier espace et le premier filtre de sorte que le contenu du premier espace est apte à venir en contact avec le premier filtre et le second filtre pour filtrer le contenu.

6. Appareil de filtre selon la revendication 4 ou 5, dans lequel l'appareil de filtre est prévu avec une partie d'insert (15) comprenant un arbre (16) raccordé, de manière mobile au premier réceptacle, l'arbre comprenant :
une partie externe s'étendant vers l'extérieur du premier réceptacle à travers une ouverture d'arbre scellée et une partie interne prévue à l'intérieur du premier espace,
un moyen d'agitation (18) pour agiter l'échantillon de graisse dans le premier espace, et
un moyen d'ouverture (19) pour se mettre en prise avec au moins une partie de l'élément de séparation pour faire passer la position d'utilisation de l'élément de séparation de la première position d'utilisation à la seconde position d'utilisation de l'élément de séparation,
dans lequel la partie externe de l'arbre est saisissable pour déplacer la partie d'insert dans la direction longitudinale de l'arbre et pour faire tourner la partie d'insert autour de la direction longitudinale de l'arbre afin d'agiter le contenu du premier espace et pour se mettre en prise avec l'élément de séparation afin de modifier sa position d'utilisation.

7. Procédé pour séparer un extrait de tissu adipeux et le tissu adipeux d'un échantillon de graisse, le procédé comprenant les étapes suivantes :
amener l'échantillon de graisse en contact avec un élément de filtre selon l'une quelconque des revendications 1 à 3, et
collecter au moins l'extrait de tissu adipeux qui a traversé l'élément de filtre.

8. Procédé selon la revendication 7, le procédé comprenant en outre les étapes suivantes :
prévoir l'échantillon de graisse dans un premier espace d'un appareil de filtre selon la revendication 5 ou 6,
modifier la position d'utilisation de l'élément de séparation pour amener l'échantillon de graisse en contact avec l'élément de filtre,
collecter l'extrait de tissu adipeux qui a traversé l'élément de filtre par le second espace, et
collecter le tissu adipeux qui n'a pas traversé l'élément de filtre par le premier espace.
